# EUROPEAN PATENT SPECIFICATION

(11) **EP 2 407 456 B1**
(45) Date of publication and mention of the grant of the patent: **28.08.2013**
(21) Application number: 09841371.9
(22) Date of filing: 24.12.2009
(51) Int. Cl.: C07D 209/86, C08F 2/46, C08F 2/50

(54) **CARBAZOLE OXIME ESTER PHOTOINITIATOR**
CARBAZOLOXIMESTERPHOTOINITIATOR
PHOTOINITIATEUR DE TYPE ESTER-OXIME DE CARBAZOLE

(30) Priority: 11.03.2009 CN 200910030326
(43) Date of publication of application: 18.01.2012
(73) Proprietor: Changzhou Tronly New Electronic Materials Co., Ltd, Jiangsu 213011 (CN)
(72) Inventor: QIAN, Xiaochun, Changzhou Jiangsu 213011 (CN)
(74) Representative: Brunetti, Fabrizio
(86) International application number: PCT/CN2009/075985
(87) International publication number: WO 2010/102502

(56) References cited:
- CN-A- 1 514 845
- CN-A- 101 508 744
- CHEN QIURONG ET AL.: 'Synthesis of a novel carbazole photoinitiator ethyl 1-(6-o-chloro-benzoyl-9-ethyl-9H-carbazol-3 yl)-ethane-1-one oxime' CHEMICAL RESEARCH AND APPLICATION vol. 20, 05 May 2008, pages 603 - 605

## Description

### FIELD OF THE INVENTION

The present invention relates to the technical field of photoinitiators and, more particularly, to a carbazole oxime ester photoinitiator and the preparation method thereof.

### BACKGROUND OF THE INVENTION

In order to make photocurable materials (photocurable coatings, ink, photoresist, CF, BM), which are comprised mainly of unsaturated resin and the monomeric material thereof, take place polymerization and solidification by irradiating them with ultraviolet rays, X-rays or laser light, photoinitiators or sensitizers must be added. By irradiating photocurable materials with ultraviolet rays, X-rays or laser light of a certain wavelength, the added photoinitiators or sensitizers can produce active groups, and initiate unsaturated groups in the photocurable materials to take place polymerization and solidification.

In photocurable materials, some traditional photoinitiators are widely used, for example, benzoin derivatives, benzil ketals, α, α'-dialkoxy acetophenones, α-hydroxy alkyl phenones, α-amino alkyl phenones, acylphosphine oxides, benzophenone/diphenylamine series, Michler's ketone, thioxanthone/ thioxanthone carboxylic amide, amine hardener, aryl diazonium salt, aryl iodonium salt, aryl sulfonium salt, ferrocene, titanocene, hexaphenylbiimidazols, triazines and traditional oxime esters and so on. Because these traditional photoinitiators have demerits more or less that sensitivity is low (both polymerization velocity and conversion are low); solubility is bad (transparency is low and there is too much photoresist residue); oxygen gas has a great effect on photo curing; storage stability is bad and so on. Therefore the use of traditional photoinitiators and photosensitive materials is considerably restricted, the performance of photosensitive materials is also greatly affected, especially, these traditional photoinitiators can not fulfill the requirements for the manufacture of BM and CF which are the key parts for new type of large-screen LCD. The emergence of a new oxime ester photoinitiator has solved above-mentioned problems to a great extent.

The photochemical performance of oxime ester compounds first emerged from the literature reported by A. Wemer and A. Piguet in Ber. Dtsch. 1904, 37, 4295; while the application of oxime ester compounds as photoinitiator first emerged from the literature reported by G. A. Delzenne, U. Laridon and H. Peeters in European Polymer Journal, 1970, 6, 933-943, the trade names are DE-OS 179508 and Agfa-Gevaert AG. The oxime ester photoinitiator which once be produced commercially and used more widely is Quantacure PDO; structural formula for Quantacure PDO is shown below.

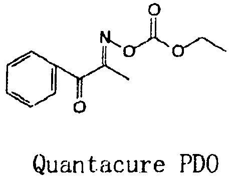

Although these traditional oxime ester photoinitiators have high initiator activity, their heat stability is low, and therefore they have been eliminated gradually. Resurrection of oxime ester photoinitiators first emerged from the literature reported by R. Mallivia et al in J. Photochem. Photobiol. A: Chemistry 2001, 138, 193 and the literature reported by L. Lavalée et al in J. Photochem. Photobiol. A: Chemistry 2002, 151, 27. Because of the introduction of diphenylsulfide and carbazolyl, which have bigger conjugated system and stronger intramolecular electronic transfer characteristics, stability and photosensitivity of the oxime ester compound are greatly improved. Two representative oxime ester photoinitiators which are widely used at present are OXE-1 and OXE-2; structural formulae for OXE-1 and OXE-2 are separately shown below.

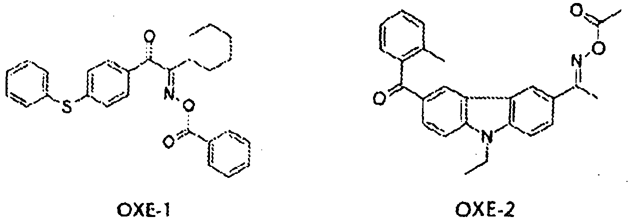

The price of BM and RGB which are mainly used in the manufacture of large-screen LCD is very high, and structural formulae for the photoinitiators has been protected by foreign companies' patents, publication numbers of which are CN 99108598 and CN 02811675.

The chemical structure of the new carbazole oxime ester photoinitiator is similar to that of OXE-2, but the practical performance (sensitivity, heat stability, solubility) of the new carbazole oxime ester photoinitiators is superior to that of OXE-2. Therefore, the new oxime ester photoinitiators can be widely used in place of OXE-1 and OXE-2 in producing the photo-initiated materials, especially in producing BM and CF, to upgrade/improve the performance thereof and meet the requirement of continuously-improved quality of related products.

### DETAILED DESCRIPTION OF THE INVENTION

Traditional oxime ester photoinitiators have the defects in practical performance. In other words, the traditional photoinitiators have lower sensitivity, heat stability and solubility. The technical problem which the inventor attempts to solve is that how to correct above-mentioned defects. It is an object of the invention to provide a new oxime ester photoinitiator which has better practical performance.

The technical solution which the inventor adopts so as to solve the above-mentioned technical problem is a carbazole oxime ester photoinitiator shown in the following general formula (I).

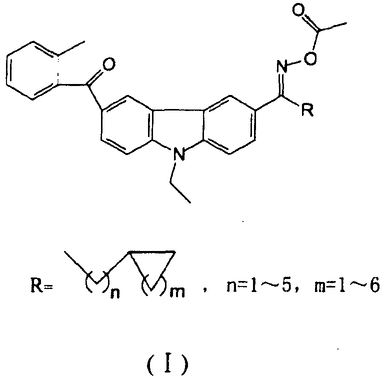

Wherein, the group R is the aliphatic hydrocarbon group substituted by the cycloalkyl, the cycloalkyl is the aliphatic cycle from cyclopropyl to cyclooctyl; the linking group between the cycloalkyl and the oxime ester group is the linear aliphatic hydrocarbon group, generally, the chain length thereof is 1-5 carbon atoms. The oxime ester compounds with this kind of chemical structure are novel compounds with excellent performance.

The process for preparing a carbazole oxime ester photoinitiator with N-ethyl carbazole as a starting material comprises the following steps:
(A) Acylation reaction: N-ethyl carbazole is acylated in organic solvent by the catalytic action of aluminium chloride to prepare an intermediate a: 3-R-acyl-6-o-methyl-benzoyl-9-ethyl carbazole; the reaction process of the acylation reaction is shown below. 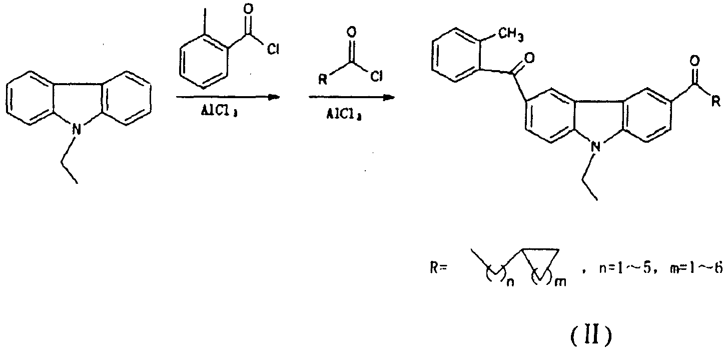 (Wherein the group R is the aliphatic hydrocarbon group substituted by the cycloalkyl, the cycloalkyl is the aliphatic cycle from cyclopropyl to cyclooctyl; the linking group between the cycloalkyl and the carbonyl is the linear aliphatic hydrocarbon group, generally, the chain length thereof is 1-5 carbon atoms)
(B) Oximation reaction: The intermediate a: 3-R-acyl-6-o-methyl-benzoyl-9-ethyl carbazole is oximated to prepare intermediate b: 1-(6-o-methyl-benzoyl-9-ethyl carbazole-3-yl)-R-one-1-oxime. The reaction process is shown below. 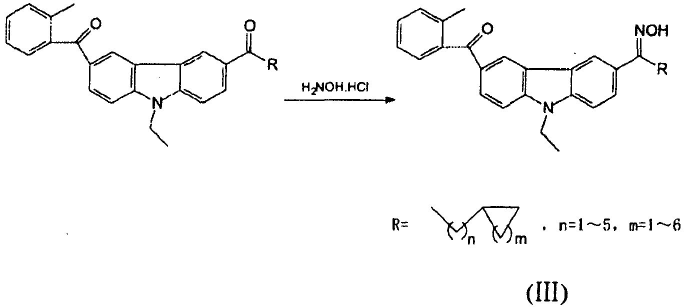 (Wherein, the group R is the aliphatic hydrocarbon group substituted by the cycloalkyl, the cycloalkyl is the aliphatic cycle from cyclopropyl to cyclooctyl; the linking group between the cycloalkyl and the oximido is the linear aliphatic hydrocarbon group, generally, the chain length thereof is 1-5 carbon atoms)
(C) Esterification reaction: The carbazole oxime ester photoinitiator is prepared by esterifying the intermediate b: 1-(6-o-methyl-benzoyl-9-ethyl carbazole-3-yl)-R-one-l-oxime with acetic anhydride; reaction process is shown below. 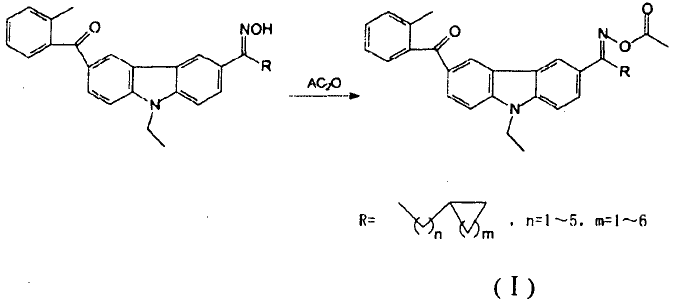 (Wherein the group R is the aliphatic hydrocarbon group substituted by the cycloalkyl, the cycloalkyl is the aliphatic cycle from cyclopropyl to cyclooctyl; the linking group between the cycloalkyl and the oxime ester group is the linear aliphatic hydrocarbon group, generally, the chain length thereof is 1-5 carbon atoms)

With respect to step A, the acylation reaction is carried out by One Pot Synthesis; reaction temperature is between 5~15°C, optimal reaction temperature is between 5~10°C; The optimal ratio (mole ratio) of N-ethyl carbazole to o-methyl-benzoyl to acyl halide is 1:1:1; Halohydrocarbons having 1-4 carbon atoms can be used as the organic solvent.

With respect to step B, making the oximeation reaction take place in water-methanol mixed solvent or water-ethanol mixed solvent is the most effective. Considering the cost of production, water-methanol mixed solvent of is optimal. The ratio (mole ratio) of intermediate a to hydroxylamine is in the range from 1:1.1 to 1:1.3, therein the optimal ratio (mole ratio) of intermediate a to hydroxylamine is 1:1.2; the optimal reaction temperature is between 76~80 °C.

With respect to step C, either acetic anhydride or acetyl chloride can be used as a reactant, but acetic anhydride is optimal; the reaction temperature is room temperature; the ratio (mole ratio) of hydroxylamine to acetyl chloride is in the range from 1:1.05 to 1:1.2, therein the optimal ratio (mole ratio) of hydroxylamine to acetyl chloride is 1:1.1.

Beneficial effects: with respect to the invention, the practical performance (sensitivity, heat stability, solubility) of the new carbazole oxime ester photoinitiator is superior to that of OXE-2, the production method is simple, industrial waste is not generated during the production process, the production cost is relatively low, and the product purity is high. Therefore, the new carbazole oxime ester photoinitiator not only can be produced in industrial-scale, but also can be prepared in laboratories.

### BRIEF DESCRIPTION OF THE DRAWINGS

Hereinafter, the invention will be concretely described with embodiments and the drawings.

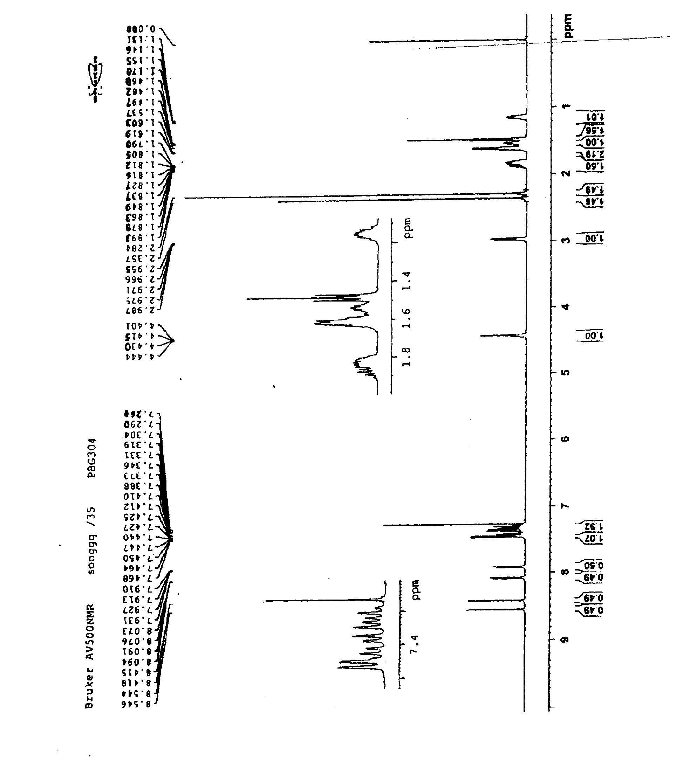

Fig. 1 is the ¹HNMR spectrogram of the sample prepared in preferred example 2 of the invention.

### DETAILED DESCRIPTION

### EXAMPLES

### Example 1:

### The process for preparing 1-(6-o-methyl-benzoyl-9-ethyl carbazole-3-yl)-3-cyclohexylacetone-1-oxime acetic acid ester

### Step 1: Preparation of 3-(3-cyclohexyl propionyl)-6-o-methyl-benzoyl-9-ethyl carbazole

Charge 39.0 g of N-ethyl carbazole, 25.3g of AlCl₃ (fine grinding) and 150 ml of dichloromethane into a 500 ml four-neck flask, carry on agitating, in order to protect the materials in the flask, pump the argon gas into the flask, cool down them with an ice bath; when the temperature falls to 0°C, begin to add dropwise the solution of 25.4 g of o-methyl-benzoyl chloride and 21 g of dichloromethane to the materials, while maintain a constant temperature of 10 °C or less, add dropwise the above-mentioned solution of o-methyl-benzoyl chloride and dichloromethane over to the materials for about 1.5 hours, continue to agitate the materials in the flask for 2 hours; then, add 25.4 g of AlCl₃ (fine grinding) to the materials; while maintaining a constant temperature of 10 °C or less, add dropwise the solution of 38.4 g of 3-cyclohexyl propionyl chloride and 20 g of dichloromethane to the materials over for about 1.5 hours; the temperature rises to 15 °C, continue to agitate the materials for 2 hours. Finally, discharge the materials.

### Post-treatment:

Under agitation, gradually add the above-mentioned materials into the diluted solution of hydrochloric acid which is prepared by mixing 65 ml of concentrated hydrochloric acid with 400 g of ice to obtain a mixture; as the materials, separate out the lower layer of the mixture with a separating funnel; as the extracted materials, extract the upper layer of the mixture with 50 ml of dichloromethane; mix the materials with the extracted materials, obtain the mixture of the two i.e. the materials and the extracted materials; in order to make the above-mentioned mixture have pH 7, wash it with the solution of sodium bicarbonate, which is prepared by mixing 10 g of sodium bicarbonate with 200 g of water, furthermore, wash it with 200 ml of water three times; subsequently, remove water with 30 g of anhydrous magnesium sulfate, remove dichloromethane via rotary evaporation method; after the evaporation, the crude product in distilling flask is a powdery solid, pour the powdery solid into 200 ml of light petroleum which is distilled at normal pressures, obtain a mixture; filtrate the mixture with air pump filtration to obtain a filter cake; pour the filter cake into 150 ml of anhydrous ethanol to obtain a mixture; heat the mixture to reflux, then, cool it down to room temperature, furthermore, freeze it for 2 hours; finally, filtrate it with air pump filtration to obtain a powdery pale yellow solid; dry the powdery pale yellow solid in an oven at 75 °C for 2 hours to obtain the intermediary product. The weight is 64.8 g; the yield is equal to 71.8 %; the purity is more than 94 %.

### Step 2: Preparation of 1-(6-o-methyl-benzoyl-9-ethyl carbazole-3-yl)-3-cyclohexylacetone-1-oxime

Charge 64.8 g of the intermediary product obtained in step 1, 12.9 g of oxammonium hydrochloride, 17.6 g of sodium acetate, 150 g of ethanol and 50 g of water into a 500 ml four-neck flask, agitate and heat them to reflux; while maintaining a constant temperature of 76°C or less, continue to agitate the materials in the flask for 5 hours; and discharge the materials. During the reaction process, large quantities of the materials were precipitated.

### Post-treatment:

Pour the above-mentioned materials into a big beaker, furthermore, pour 1000 g of water into the same beaker, carry on agitating to obtain a mixture; leave the mixture at rest overnight; filtrate the above-mentioned mixture with air pump filtration to obtain a powdery white solid; pour the powdery white solid into tetrahydrofuran (THF) to obtain a mixture; in order to remove water from the mixture, pour 50 g of anhydrous magnesium sulfate into it; carry on filtrating to obtain a filtrate; evaporate the filtrate via rotary evaporation method; after the evaporation, oily viscous material is obtained in distilling flask, pour the oily viscous material into 150 ml of anhydrous methanol, carry on agitating to obtain a precipitate; filtrate the precipitate with air pump filtration to obtain a powdery white solid, dry the powdery white solid in an oven at 70 °C for 5 hours to obtain the intermediary product. The weight is 54.2 g; the yield is equal to 81 %; and the purity is more than 95 %.

### Step 3: Preparation of 1-(6-o-methyl-benzoyl-9-ethyl carbazole-3-yl)-3-cyclohexylacetone-1-oxime acetic acid ester

Charge 54.2 g of the intermediary product obtained in step 2, 350 ml of dichloromethane and 15.3 g of triethyl amine into a 1000 ml four-neck flask, carry on agitating, cool down the materials in the flask with an ice bath; when the temperature falls to 0 °C, begin to add dropwise the solution of 9.2 g of acetyl chloride and 30 g of dichloromethane to the materials for about 1.5 hours; continue to agitate the materials for 1 hours; then begin to add dropwise 500 ml of cold water to the materials; separate out organic liquid layer of the materials with a separating funnel, in order to make the organic liquid layer have pH 7, wash it with 200 ml of 5% sodium bicarbonate one time, furthermore, wash it with 200 ml of water two times; then wash it with the diluted solution of hydrochloric acid one time, which is prepared by mixing 20 g of concentrated hydrochloric acid with 400 ml of water, furthermore, wash it with 200 ml of water three times; subsequently, in order to remove water, pour 100 g of anhydrous magnesium sulfate into it; distill out the solvents via rotary evaporation method to obtain a viscous liquid; dissolve the viscous liquid in methanol to obtain the mixed solution of viscous liquid-methanol; under high-speed agitation, add dropwise the mixed solution of viscous liquid-methanol to larger quantities of water to obtain a white solid product as a precipitate; finally, carry on filtrating and drying to obtain a final product. The weight is 51.9 g; the yield is equal to 88 %; and the purity is more than 95 %. Structural formula of the white solid product is shown below.

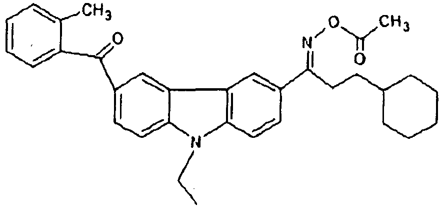

### Example 2:

### The process for preparing 1-(6-o-methyl-benzoyl-9-ethyl carbazole-3-yl)-3-cyclopentylacetone-1-oxime acetic acid ester

### Step 1: Preparation of 3-(3-cyclopentyl propionyl)-6-o-methyl-benzoyl-9-ethyl carbazole

Charge 39.0 g of N-ethyl carbazole, 25.3g of AlCl₃ (fine grinding) and 150 ml of dichloromethane into a 500 ml four-neck flask, carry on agitating, in order to protect the materials in the flask, pump the argon gas into the flask, cool down them with an ice bath; when the temperature falls to 0 °C, begin to add dropwise the solution of 25.4 g of o-methyl-benzoyl chloride and 21 g of dichloromethane to the materials, while maintain a constant temperature of 10 °C or less, add dropwise the above-mentioned solution of o-methyl-benzoyl chloride and dichloromethane over to the materials for about 1.5 hours, continue to agitate the materials in the flask for 2 hours; then add 25.4 g of AlCl₃ (fine grinding) to the materials; while maintaining a constant temperature of 10 °C or less, add dropwise the solution of 42.2 g of 3- cyclopentyl propionyl chloride and 20 g of dichloromethane to the materials over for about 1.5 hours; the temperature rises to 15 °C, continue to agitate the materials for 2 hours. Finally, discharge the materials.

### Post-treatment:

Under agitation, gradually add the above-mentioned materials into the diluted solution of hydrochloric acid, which is prepared by mixing 65 ml of concentrated hydrochloric acid with 400 g of ice to obtain a mixture; as the materials, separate out the lower layer of the mixture with a separating funnel; as the extracted materials, extract the upper layer of the mixture with 50 ml of dichloromethane; mix the materials with the extracted materials, obtain the mixture of the two i.e. the materials and the extracted materials; in order to make the above-mentioned mixture have pH 7, wash it with the solution of sodium bicarbonate, which is prepared by mixing 10 g of sodium bicarbonate with 200 g of water, furthermore, wash it with 200 ml of water three times; subsequently, remove water with 30 g of anhydrous magnesium sulfate, remove dichloromethane via rotary evaporation method; after the evaporation, the crude product in distilling flask is a powdery solid, pour the powdery solid into 200 ml of light petroleum which is distilled at normal pressures to obtain a mixture; filtrate the mixture with air pump filtration to obtain a filter cake; pour the filter cake into 150 ml of anhydrous ethanol to obtain a mixture; heat the mixture to reflux, then, cool it down to room temperature, furthermore, freeze it for 2 hours; finally, filtrate it with air pump filtration to obtain a powdery pale yellow solid, dry the powdery pale yellow solid in an oven at 75 °C for 2 hours to obtain the intermediary product. The weight is 50.3 g; the purity is more than 96 %.

### Step 2: Preparation of 1-(6-o-methyl-benzoyl-9-ethyl carbazole-3-yl)-3-cyclopentylacetone-1-oxime

Charge 44.5 g of the intermediary product obtained in step 1, 9.7 g of oxammonium hydrochloride, 13 g of sodium acetate, 150 g of ethanol and 50 g of water into a 500 ml four-neck flask, agitate and heat them to reflux; while maintaining a constant temperature of 10 °C or less, continue to agitate the materials in the flask for 5 hours; discharge the materials. During the reaction process, large quantities of the materials were precipitated.

### Post-treatment:

Pour the above-mentioned materials into a big beaker, furthermore, pour 1000 g of water into the same beaker, carry on agitating to obtain a mixture; leave the mixture at rest overnight; filtrate the above-mentioned mixture with air pump filtration to obtain a powdery white solid; pour the powdery white solid into tetrahydrofuran (THF) to obtain a mixture; in order to remove water from the mixture, pour 50 g of anhydrous magnesium sulfate into it; carry on filtrating to obtain a filtrate; evaporate the filtrate via rotary evaporation method; after the evaporation, oily viscous material is obtained in distilling flask, pour the oily viscous material into 150 ml of anhydrous methanol, carry on agitating to obtain a precipitate; filtrate the precipitate with air pump filtration to obtain a powdery white solid, dry the powdery white solid in an oven at 70 °C for 5 hours to obtain the intermediary product. The weight is 37.6 g; the yield is equal to 80.0 %; and the purity is more than 95.0 %.

### Step 3: Preparation of 1-(6-o-methyl-benzoyl-9-ethyl carbazole-3-yl)-3-cyclopentylacetone-1-oxime acetic acid ester

Charge 86 g of the intermediary product obtained in step 2, and 270 ml of dichloromethane into a 500 ml four-neck flask, carry on agitating, at normal temperature, begin to add dropwise the solution of 32 g of acetic anhydride and 32 g of dichloromethane to the materials for about 1.5 hours, continue to agitate the materials for 1 hours; then begin to add dropwise 260 g of 5% sodium hydroxide to the materials; after reaction, discharge the materials; separate out the organic liquid layer of the materials with a separating funnel, in order to make the organic liquid layer have pH 7, wash it with water three times; distill out dichloromethane via rotary evaporation method to obtain a viscous liquid; dissolve the viscous liquid in 400 ml of methanol to obtain the mixed solution of viscous liquid-methanol; under high-speed agitation, add dropwise the mixed solution of viscous liquid-methanol to larger quantities of water to obtain a white solid product as a precipitate; finally, carry on filtrating and drying to obtain a final product. Carry on instrumental analysis of the final product. The weight is 95.0 g; the purity is more than 98 %; ¹HNMR spectrogram is shown in Fig. 1, the structural formula is shown below.

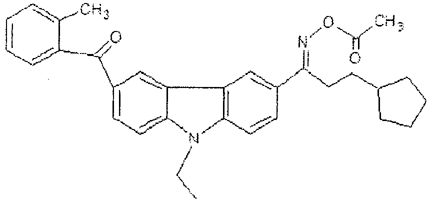

Those skilled in the art can take hints from the preferred embodiments of the invention according to the contents as described hereinabove, and carry on various changing and modifying without departing from the scope of the invention.

## Claims

1. A carbazole oxime ester photoinitiator shown in general formula (I), 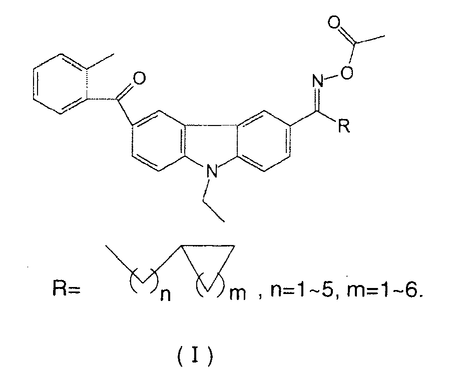 wherein the group R is the aliphatic hydrocarbon group substituted by the cycloalkyl, the cycloalkyl is the aliphatic cycle from cyclopropyl to cyclooctyl; the linking group between the cycloalkyl and the oxime ester group is the linear aliphatic hydrocarbon group, generally, the chain length thereof is 1-5 carbon atoms.

2. A process for preparing the carbazole oxime ester photoinitiator according to claim 1 where N-ethyl carbazole is used, **characterized in that** it comprises the following steps:
A. Acylation reaction: intermediate a: 3-R-acyl-6-o-methyl-benzoyl-9-ethyl carbazole is prepared by acylating N-ethyl carbazole in organic solvent by the catalytic action of AlCl₃;
B. Oximeation reaction: intermediate b: 1-(6-o-methyl-benzoyl-9-ethyl carbazole-3-yl)-R-one-1-oxime is prepared synthetically from the intermediate a;
C. Esterification reaction: a carbazole oxime ester photoinitiator is prepared by esterifying the 1-(6-o-methyl-benzoyl-9-ethyl carbazole-3-yl)-R-one-1-oxime with acetic anhydride.

3. A process for preparing the carbazole oxime ester photoinitiator according to claim 2, **characterized in that** the structural formula for the intermediate a: 3-R-acyl-6-o-methyl-benzoyl-9-ethyl carbazole which is prepared in acylation reaction of step A is shown below, 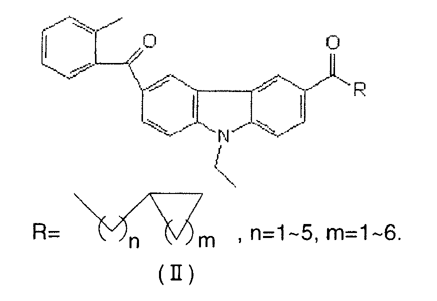 wherein the group R is the aliphatic hydrocarbon group substituted by the cycloalkyl, the cycloalkyl is the aliphatic cycle from cyclopropyl to cyclooctyl; the linking group between the cycloalkyl and the carbonyl is the linear aliphatic hydrocarbon group, generally, the chain length thereof is 1-5 carbon atoms.

4. A process for preparing the carbazole oxime ester photoinitiator according to claim 2, **characterized in that** the organic solvent which is used in acylation reaction of step A is halohydrocarbon having 1-4 carbon atoms.

5. A process for preparing the carbazole oxime ester photoinitiator according to claim 2, **characterized in that** the structural formula for the intermediate b: 1-(6-o-methyl-benzoyl-9-ethyl carbazole-3-yl)-R-one-1-oxime which is prepared in acylation reaction of step B is shown below, 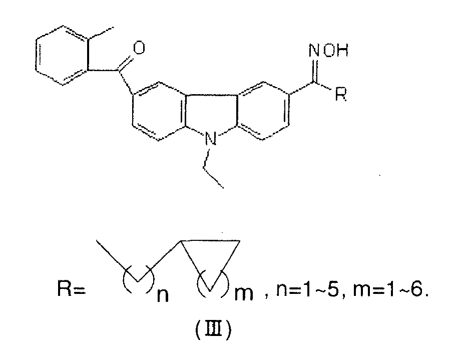 wherein the group R is the aliphatic hydrocarbon group substituted by the cycloalkyl, the cycloalkyl is the aliphatic cycle from cyclopropyl to cyclooctyl; the linking group between the cycloalkyl and the oximido is the linear aliphatic hydrocarbon group, generally, the chain length thereof is 1-5 carbon atoms.

6. A carbazole oxime ester photoinitiator according to claim 1, shown in general formula (Ia), 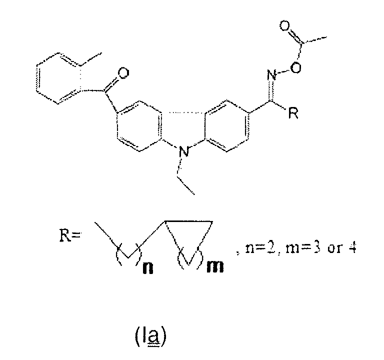 wherein the group R is the aliphatic hydrocarbon group substituted by the cycloalkyl, the cycloalkyl is cyclopentyl or cyclohexyl; the linking group between the cycloalkyl and the oxime ester group is the linear aliphatic hydrocarbon group, generally, the chain length thereof is 2 carbon atoms.

7. A process for preparing the carbazole oxime ester photoinitiator according to claim 6 where N-ethyl carbazole is used, **characterized in that** it comprises the following steps:
A. Acylation reaction: intermediate a: 3-R-acyl-6-o-methyl-benzoyl-9-ethyl carbazole is prepared by acylating N-ethyl carbazole in organic solvent by the catalytic action of AlCl₃;
B. Oximeation reaction: intermediate b: 1-(6-o-methyl-benzoyl-9-ethyl carbazole-3-yl)-R-one-1-oxime is prepared synthetically from the intermediate a;
C. Esterification reaction: a carbazole oxime ester photoinitiator is prepared by esterifying the 1-(6-o-methyl-benzoyl-9-ethyl carbazole-3-yl)-R-one-1-oxime with acetic anhydride.

8. A process for preparing the carbazole oxime ester photoinitiator according to claim 7, **characterized in that** the structural formula for the intermediate a: 3-R-acyl-6-o-methyl-benzoyl-9-ethyl carbazole which is prepared in acylation reaction of step A is shown below, 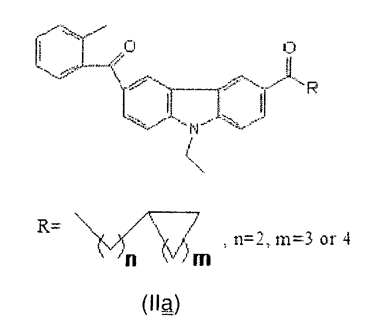 wherein the group R is the aliphatic hydrocarbon group substituted by the cycloalkyl, the cycloalkyl is cyclopentyl or cyclohexyl; the linking group between the cycloalkyl and the carbonyl is the linear aliphatic hydrocarbon group, generally, the chain length thereof is 2 carbon atoms.

9. A process for preparing the carbazole oxime ester photoinitiator according to claim 7, **characterized in that** the organic solvent which is used in acylation reaction of step A is halohydrocarbon having 1-4 carbon atoms.

10. A process for preparing the carbazole oxime ester photoinitiator according to claim 7, **characterized in that** the structural formula for the intermediate b: 1-(6-o-methyl-benzoyl-9-ethyl carbazole-3-yl)-R-one-1-oxime which is prepared in acylation reaction of step B is shown below, 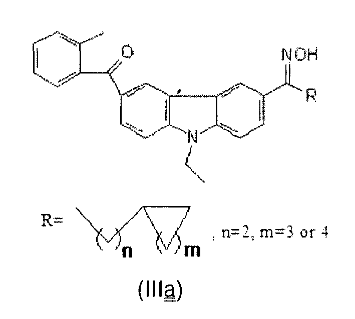 wherein the group R is the aliphatic hydrocarbon group substituted by the cycloalkyl, the cycloalkyl is cyclopentyl or cyclohexyl; the linking group between the cycloalkyl and the oximido is the linear aliphatic hydrocarbon group, generally, the chain length thereof is 2 carbon atoms.

## Patentansprüche

1. Carbazoloximesterphotoinitiator, der durch die allgemeine Formel (I) dargestellt wird: 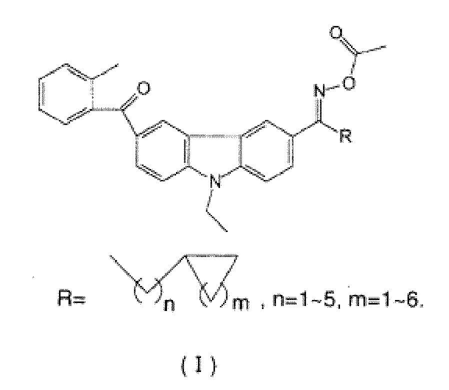 wobei die Gruppe R die aliphatische Kohlenwasserstoffgruppe ist, die durch das Cycloalkyl substituiert ist, das Cycloalkyl der aliphatische Zyklus von Cyclopropyl bis Cyclooctyl ist, die Kopplungsgruppe zwischen dem Cycloalkyl und der Oximestergruppe die lineare aliphatische Kohlenwasserstoffgruppe ist und im allgemeinen die Kettenlänge davon 1 bis 5 Kohlenstoffatome beträgt.

2. Verfahren zum Herstellen des Carbazoloximesterphotoinitiators nach Anspruch 1, wobei N-Ethylcarbazol verwendet wird, **dadurch gekennzeichnet, daß** es die folgenden Schritte umfaßt:
A) Acylierungsreaktion: Zwischenprodukt a: 3-R-Acyl-6-o-methyl-benzoyl-9-ethylcarbazol wird durch Acylierung des N-Ethylcarbazols in einem organischen Lösungsmittel durch die katalytische Wirkung von AlCl₃ hergestellt,
B) Oximationsreaktion: Zwischenprodukt b: 1-(6-o-Methyl-benzoyl-9-ethylcarbazol-3-yl)-R-on-1-oxim wird synthetisch aus dem Zwischenprodukt a hergestellt,
C) Veresterungsreaktion: ein Carbazoloximesterphotoinitiator wird durch Verestern des 1-(6-o-Methyl-benzoyl-9-ethylcarbazol-3-yl)-R-on-1-oxim mit Essigsäureanhydrid hergestellt.

3. Verfahren zum Herstellen des Carbazoloximesterphotoinitiators nach Anspruch 2, **dadurch gekennzeichnet, daß** die Strukturformel für das Zwischenprodukt a: 3-R-Acyl-6-o-methyl-benzoyl-9-ethylcarbazol, das in der Acylierungsreaktion in Schritt A hergestellt wird, unten gezeigt ist: 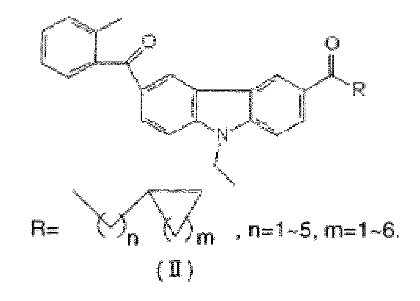 wobei die Gruppe R die aliphatische Kohlenwasserstoffgruppe ist, die durch das Cycloalkyl substituiert ist, das Cycloalkyl der aliphatische Zyklus von Cyclopropyl bis Cyclooctyl ist, die Kopplungsgruppe zwischen dem Cycloalkyl und dem Carbonyl die lineare aliphatische Kohlenwasserstoffgruppe ist und im allgemeinen die Kettenlänge davon 1 bis 5 Kohlenstoffatome beträgt.

4. Verfahren zum Herstellen des Carbazoloximesterphotoinitiators nach Anspruch 2, **dadurch gekennzeichnet, daß** das organische Lösungsmittel, das in der Acylierungsreaktion in Schritt A verwendet wird, ein Halokohlenwasserstoff mit 1 bis 4 Kohlenstoffatomen ist.

5. Verfahren zum Herstellen des Carbazoloximesterphotoinitiators nach Anspruch 2, **dadurch gekennzeichnet, daß** die Strukturformel für das Zwischenprodukt b: 1-(6-o-Methyl-benzoyl-9-ethylcarbazol-3-yl)-R-on-1-oxim, das in der Acylierungsreaktion in Schritt B hergestellt wird, unten gezeigt ist: 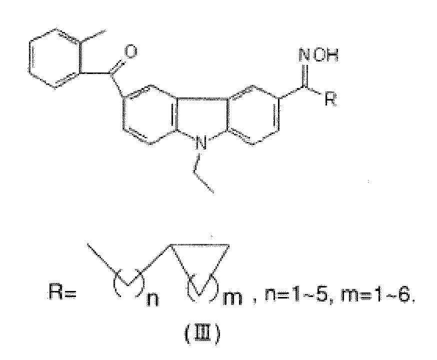 wobei die Gruppe R die aliphatische Kohlenwasserstoffgruppe ist, die durch das Cycloalkyl substituiert ist, das Cycloalkyl der aliphatische Zyklus von Cyclopropyl bis Cyclooctyl ist, die Kopplungsgruppe zwischen dem Cycloalkyl und dem Oximido die lineare aliphatische Kohlenwasserstoffgruppe ist und im allgemeinen die Kettenlänge davon 1 bis 5 Kohlenstoffatome beträgt.

6. Carbazoloximesterphotoinitiator nach Anspruch 1, der durch die allgemeine Formel (la) dargestellt wird: 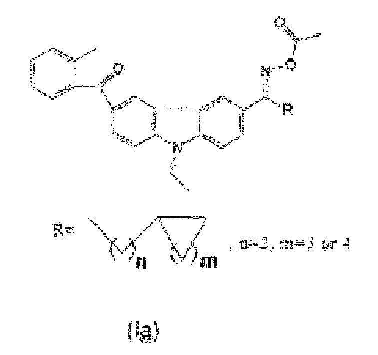 wobei die Gruppe R die aliphatische Kohlenwasserstoffgruppe ist, die durch das Cycloalkyl substituiert ist, das Cycloalkyl Cyclopentyl oder Cyclohexyl ist, die Kopplungsgruppe zwischen dem Cycloalkyl und der Oximestergruppe die lineare aliphatische Kohlenwasserstoffgruppe ist und im allgemeinen die Kettenlänge davon 2 Kohlenstoffatome beträgt.

7. Verfahren zum Herstellen des Carbazoloximesterphotoinitiators nach Anspruch 6, wobei N-Ethylcarbazol verwendet wird, **dadurch gekennzeichnet, daß** es die folgenden Schritte umfaßt:
A) Acylierungsreaktion: Zwischenprodukt a: 3-R-Acyl-6-o-methyl-benzoyl-9-ethylcarbazol wird durch Acylierung des N-Ethylcarbazols in einem organischen Lösungsmittel durch die katalytische Wirkung von AlCl₃ hergestellt,
B) Oximationsreaktion: Zwischenprodukt b: 1-(6-o-Methyl-benzoyl-9-ethylcarbazol-3-yl)-R-on-1-oxim wird synthetisch aus dem Zwischenprodukt a hergestellt,
C) Veresterungsreaktion: ein Carbazoloximesterphotoinitiator wird durch Verestern des 1-(6-o-Methyl-benzoyl-9-ethylcarbazol-3-yl)-R-on-1-oxim mit Essigsäureanhydrid hergestellt.

8. Verfahren zum Herstellen des Carbazoloximesterphotoinitiators nach Anspruch 7, **dadurch gekennzeichnet, daß** die Strukturformel für das Zwischenprodukt a: 3-R-Acyl-6-o-methyl-benzoyl-9-ethylcarbazol, das in der Acylierungsreaktion in Schritt A hergestellt wird, unten gezeigt ist: 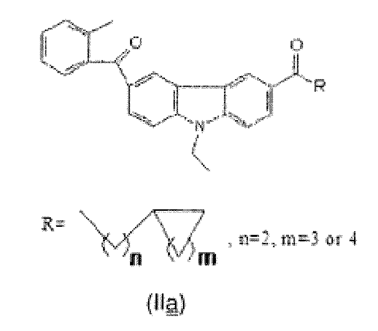 wobei die Gruppe R die aliphatische Kohlenwasserstoffgruppe ist, die durch das Cycloalkyl substituiert ist, das Cycloalkyl Cyclopentyl oder Cyclohexyl ist, die Kopplungsgruppe zwischen dem Cycloalkyl und dem Carbonyl die lineare aliphatische Kohlenwasserstoffgruppe ist und im allgemeinen die Kettenlänge davon 2 Kohlenstoffatome beträgt.

9. Verfahren zum Herstellen des Carbazoloximesterphotoinitiators nach Anspruch 7, **dadurch gekennzeichnet, daß** das organische Lösungsmittel, das in der Acylierungsreaktion in Schritt A verwendet wird, ein Halokohlenwasserstoff mit 1 bis 4 Kohlenstoffatomen ist.

10. Verfahren zum Herstellen des Carbazoloximesterphotoinitiators nach Anspruch 7, **dadurch gekennzeichnet, daß** die Strukturformel für das Zwischenprodukt b: 1-(6-o-Methyl-benzoyl-9-ethylcarbazol-3-yl)-R-on-1-oxim, das in der Acylierungsreaktion in Schritt B hergestellt wird, unten gezeigt ist: 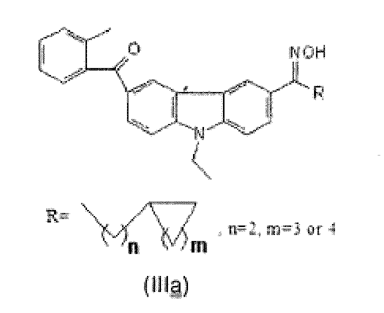 wobei die Gruppe R die aliphatische Kohlenwasserstoffgruppe ist, die durch das Cycloalkyl substituiert ist, das Cycloalkyl Cyclopentyl oder Cyclohexyl ist, die Kopplungsgruppe zwischen dem Cycloalkyl und dem Oximido die lineare aliphatische Kohlenwasserstoffgruppe ist und im allgemeinen die Kettenlänge davon 2 Kohlenstoffatome beträgt.

## Revendications

1. Photo-initiateur ester de carbazole-oxime montré dans la formule générale (I), 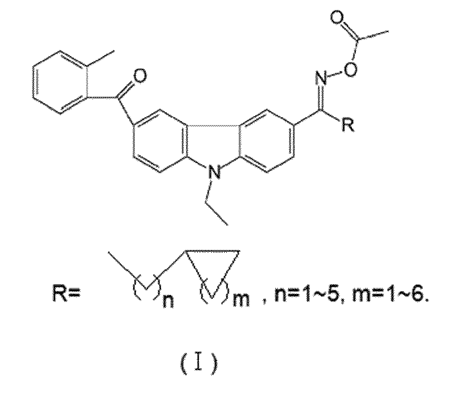 où le groupe R est le groupe hydrocarboné aliphatique substitué par le cycloalkyle, le cycloalkyle est le cycle aliphatique de cyclopropyle à cyclooctyle ; le groupe de liaison entre le cycloalkyle et le groupe ester d'oxime est le groupe hydrocarboné aliphatique linéaire, généralement, sa longueur de chaîne est 1-5 atomes de carbone.

2. Procédé pour préparer le photo-initiateur ester de carbazole-oxime selon la revendication 1 où le N-éthylcarbazole est utilisé, **caractérisé en ce qu'**il comprend les étapes suivantes :
A. Réaction d'acylation : intermédiaire a : le 3-R-acyl-6-o-méthyl-benzoyl-9-éthyl carbazole est préparé par acylation du N-éthyl carbazole dans un solvant organique par l'action catalytique de AlCl₃ ;
B. Réaction d'oximation : intermédiaire b : la 1-(6-o-méthyl-benzoyl-9-éthyl carbazol-3-yl)-R-one-1-oxime est préparée par synthèse à partir de l'intermédiaire a ;
C. Réaction d'estérification : un photo-initiateur ester de carbazole-oxime est préparé par estérification de la 1-(6-o-méthyl-benzoyl-9-éthyl carbazol-3-yl)-R-one-1-oxime avec l'anhydride acétique.

3. Procédé pour préparer le photo-initiateur ester de carbazole-oxime selon la revendication 2, **caractérisé en ce que** la formule structurale pour l'intermédiaire a : 3-R-acyl-6-o-méthyl-benzoyl-9-éthyl carbazole qui est préparé dans la réaction d'acylation de l'étape A est montrée ci-dessous, 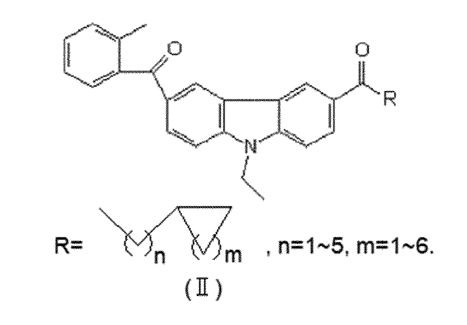 où le groupe R est le groupe hydrocarboné aliphatique substitué par le cycloalkyle, le cycloalkyle est le cycle aliphatique de cyclopropyle à cyclo-octyle ; le groupe de liaison entre le cycloalkyle et le carbonyle est le groupe hydrocarboné aliphatique linéaire, généralement, sa longueur de chaîne est 1-5 atomes de carbone.

4. Procédé pour préparer le photo-initiateur ester de carbazole-oxime selon la revendication 2, **caractérisé en ce que** le solvant organique qui est utilisé dans la réaction d'acylation de l'étape A est un halohydrocarbure ayant 1-4 atomes de carbone.

5. Procédé pour préparer le photo-initiateur ester de carbazole-oxime selon la revendication 2 **caractérisé en ce que** la formule structurale pour l'intermédiaire b : 1-(6-o-méthyl-benzoyl-9-éthyl carbazol-3-yl)-R-one-1-oxime qui est préparé dans la réaction d'acylation de l'étape B est montrée ci-dessous 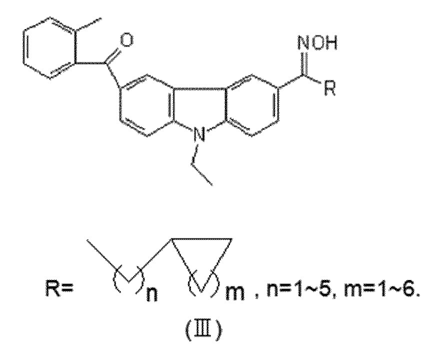 où le groupe R est le groupe hydrocarboné aliphatique substitué par le cycloalkyle, le cycloalkyle et le cycle aliphatique de cyclopropyle à cyclo-octyle ; le groupe de liaison entre le cycloalkyle et l'oximido est le groupe hydrocarboné aliphatique linéaire, généralement, sa longueur de chaîne est 1-5 atomes de carbone.

6. Photo-initiateur ester de carbazole-oxime selon la revendication 1 montré dans la formule générale (Ia), 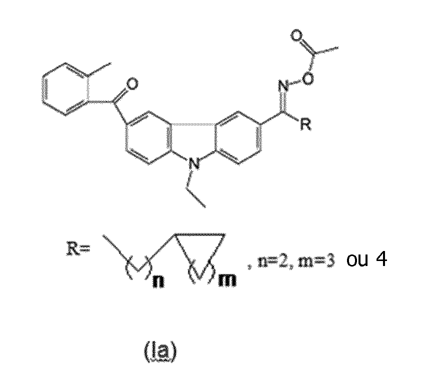 où le groupe R est le groupe hydrocarboné aliphatique substitué par le cycloalkyle, le cycloalkyle est cyclopentyle ou cyclohexyle ; le groupe de liaison entre le cycloalkyle et le groupe ester d'oxime est le groupe hydrocarboné aliphatique linéaire, généralement sa longueur de chaîne est 2 atomes de carbone.

7. Procédé pour préparer le photo-initiateur ester de carbazole-oxime selon la revendication 6, où le N-éthylcarbazole est utilisé, **caractérisé en ce qu'**il comprend les étapes suivantes :
A. Réaction d'acylation : intermédiaire a : le 3-R-acyl-6-o-méthyl-benzoyl-9-éthyl carbazole est préparé par acylation du N-éthylcarbazole dans un solvant organique par l'action catalytique de AlCl₃;
B. Réaction d'oximation : intermédiaire b : la 1-(6-o-méthyl-benzoyl-9-éthyl carbazol-3-yl)-R-one-1-oxime est préparée par synthèse à partir de l'intermédiaire a ;
C. Réaction d'estérification : un photo-initiateur ester de carbazole-oxime est préparé par estérification de la 1-(6-o-méthyl-benzoyl-9-éthyl carbazol-3-yl)-R-one-1-oxime avec l'anhydride acétique.

8. Procédé pour préparer le photo-initiateur ester de carbazole-oxime selon la revendication 7, **caractérisé en ce que** la formule structurale pour l'intermédiaire a : 3-R-acyl-6-o-méthyl-benzoyl-9-éthyl carbazole qui est préparé dans la réaction d'acylation de l'étape A est montrée ci-dessous, 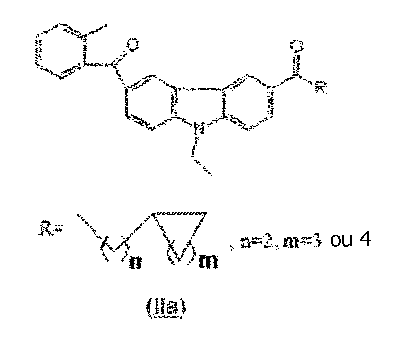 où le groupe R est le groupe hydrocarboné aliphatique substitué par le cycloalkyle, le cycloalkyle est cyclopentyle ou cyclohexyle; le groupe de liaison entre le cycloalkyle et le carbonyle est le groupe hydrocarboné aliphatique linéaire, généralement, sa longueur de chaîne est 2 atomes de carbone.

9. Procédé pour préparer le photo-initiateur ester de carbazole-oxime selon la revendication 7, **caractérisé en ce que** le solvant organique qui est utilisé dans la réaction d'acylation de l'étape A est un halohydrocarbure ayant 1-4 atomes de carbone.

10. Procédé pour préparer le photo-initiateur ester de carbazole-oxime selon la revendication 7, **caractérisé en ce que** la formule structurale pour l'intermédiaire b : 1-(6-o-méthyl-benzoyl-9-éthyl carbazol-3-yl)-R-one-1-oxime qui est préparé dans la réaction d'acylation de l'étape B est montrée ci-dessous, 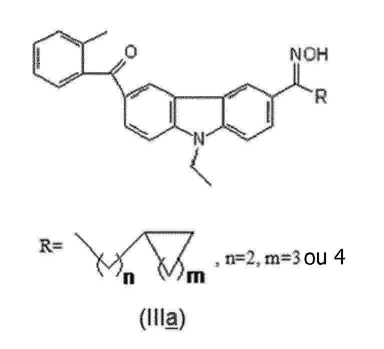 où le groupe R est le groupe hydrocarboné aliphatique substitué par le cycloalkyle, le cycloalkyle est cyclopentyle ou cyclohexyle; le groupe de liaison entre le cycloalkyle et l'oximido est le groupe hydrocarboné aliphatique linéaire, généralement, sa longueur de chaîne est 2 atomes de carbone.
